# EUROPEAN PATENT APPLICATION

(11) **EP 4 108 225 A1**
(43) Date of publication of application: **28.12.2022**
(21) Application number: 22180493.3
(22) Date of filing: 22.06.2022
(51) Int. Cl.: A61K 8/11, A61K 8/35, A61K 8/73, A61K 8/9706, A61Q 19/00, A61K 8/66

(54) **METHOD FOR PRODUCING MICRO/NANO CAPSULES CONTAINING EXTRACTS OF MICROALGAE AND/OR CYANOBACTERIA FOR COSMETIC USE**

(30) Priority: 23.06.2021 IT 202100016454
(71) Applicant: Nanomnia Srl, 37059 Perzacco di Zevio (VR) (IT)
(72) Inventor: BOVI, Michele, 37100 VERONA (IT); VACCARI, Pietro, 37100 VERONA (IT); FACCIOTTI, Camilla, 37014 CASTELNUOVO DEL GARDA (VR) (IT); BONACONSA, Marta, 37059 PERZACCO DI ZEVIO (VR) (IT)
(74) Representative: De Sandre, Emanuele

(57) **Abstract**

Method for producing cosmetic micro/nano capsules containing at least one antioxidant compound preferably extracted from microalgae and/or cyanobacteria, characterized in that it comprises the following steps:
a) solubilising the antioxidant compound in an organic solvent;
b) mixing the solution obtained in step a) with an aqueous solution of lysozyme to obtain an antioxidant complex formed by the antioxidant compound and by the lysozyme;
c) evaporating at least a part of the organic solvent present in the solution obtained in step b);
d) mixing the solution obtained in step c) with an aqueous solution of hyaluronic acid;
e) homogenising the solution obtained in step d) to produce a homogeneous suspension of the antioxidant complex;
f) adding to the homogeneous suspension obtained in step e) an aqueous solution of a cross-linking compound and performing the homogenisation of the aqueous solution of a cross-linking compound in the homogeneous suspension obtained in step e) to produce the micro/nano capsules containing the antioxidant compound.

## Description

The present invention concerns a method for producing micro/nano capsules containing antioxidant compounds, preferably antioxidant compounds extracted from microalgae and/or cyanobacteria, for cosmetic use.

The present invention further concerns a cosmetic formulation comprising the micro/nano capsules made with the aforesaid method.

### BACKGROUND

Microalgae and cyanobacteria represent a heterogeneous group of photosynthetic microorganisms capable of biosynthesising, accumulating and secreting numerous types of primary and secondary metabolites in response to environmental changes and biotic and abiotic stresses of various type.

Some of these metabolites are particularly beneficial for use in industrial, medical and cosmetic applications.

In particular, some of these metabolites are able to stimulate the ability of cells to self-repair or protect themselves against external stresses of various type, such as for example solar radiation, heat, physical and chemical stresses.

For example, astaxanthin is a carotenoid produced in large quantities by the freshwater microalgae *Haematococcus pluvialis,* which produces it mixed with lipids when environmental conditions induce oxidative stress on the microalgae, such as in the presence of salt water or high light radiation. Astaxanthin has excellent antioxidant and anti-inflammatory properties that make it suitable for use in reducing oxidative stresses such as protein and lipid oxidation and DNA damage.

Disadvantageously, astaxanthin is particularly unstable to heat, light radiation and presence of oxygen.

Additionally, this compound is substantially insoluble in water.

For these reasons, the applications of astaxanthin are currently limited.

In particular, this compound cannot be used as an active ingredient in formulations for topical use due to its chemical instability and its insolubility in water, which decrease its absorption in the skin, thus reducing its bioavailability.

Phycocyanin represents an association of proteins found in *Cyanobacteria* of the species *Arthrospira platensis* and *Rhodophyta.*

It comprises proteins belonging to the phycobiliprotein family, and water-soluble photosynthesis pigments called phycocyanobilins.

Phycocyanin is particularly beneficial as it has excellent antioxidant, anti-inflammatory, anti-cancer and fibroblast migration-stimulating properties.

In addition, the phycocyanobilin pigment is responsible for the intense blue colour of this protein, which would seemingly be able to prevent damage from oxidative stress.

Unlike astaxanthin, phycocyanin is water-soluble, however, it is particularly unstable to environmental stresses such as heat and solar radiation, again reducing its possible applications in medicine and cosmetics.

The document "Enzymatically crosslinked hyaluronic acid microgels as a vehicle for sustained delivery of cationic proteins" by Elaheh Jooybar et al. describes a method for preparing a hyaluronic acid-based microgel using the enzyme horseradish peroxidase HRP. This enzyme is an antioxidant compound. However, its function in the aforesaid method of prior art is to catalyse the cross-linking reaction of the hyaluronic acid chains in order to promote the formation of the microgel, within which cationic proteins will then be incorporated. In fact, HRP, thanks to its good solubility in water, is dissolved in the aqueous solvent PBS so as to be in contact with the hyaluronic acid chains, which in turn are water-soluble, and to exercise its cross-linking function. Although not explicitly stated in the above-mentioned document of prior art, it is believed that, at the end of the preparation of the aforesaid microgel, HRP is removed from the reaction environment due to the precipitation reaction of the microgel particles in acetone. HRP is in fact poorly soluble in organic solvents. In any case, even if HRP remained incorporated in the microgel meshes, it is likely that the presence of the organic solvent causes the decrease in the alpha-helix structure content and the modification of its tertiary structure at the aromatic amino acid residues (*A*kita M. et al., Biosci. Biotechnol. Biochem. 65 (7), 1581-1588, 2001)*,* thus decreasing its antioxidant activity.

In light of the above, it is clear that it is necessary to identify new methods for producing cosmetic formulations, and therefore new cosmetic formulations, containing natural antioxidant compounds that overcome the aforesaid drawbacks and can also be used for topical use.

In particular, there is a need for new antioxidant cosmetic formulations with one or more improved characteristics compared to the antioxidant cosmetic formulations currently available.

It is therefore an object of the present invention to provide a new method for producing cosmetic formulations comprising antioxidants of natural origin, in particular antioxidants extracted from microalgae and/or cyanobacteria.

It is also the object of the present invention that this method makes it possible to produce formulations in which the active ingredients present in them are protected from environmental stresses, such as heat, light radiation and, in some cases, the presence of oxygen, in such a way that the antioxidant function of these active ingredients is maintained unaltered.

Furthermore, it is an object of the present invention that the active ingredients present in such formulations exhibit excellent bioavailability, in particular for topical use.

Last but not least, it is an object of the present invention to provide a cosmetic formulation made with the aforesaid method.

The aforesaid objects are achieved by a method for producing micro/nano capsules containing at least one antioxidant compound preferably extracted from microalgae and/or cyanobacteria, for cosmetic use, according to independent claim 1.

The objects are further achieved by a cosmetic formulation, as set forth in claim 10, by a device for the application of cosmetics topically according to claim 12 and by the use of said cosmetic formulation for producing, by three-dimensional printing, a device for the application of cosmetics topically, as set forth in claim 13.

Further features of the invention are described in the dependent claims.

The features and advantages of the present invention will be evident from the detailed description below, and from the examples provided by way of illustration and not limitation.

### BRIEF DESCRIPTION OF THE DRAWINGS

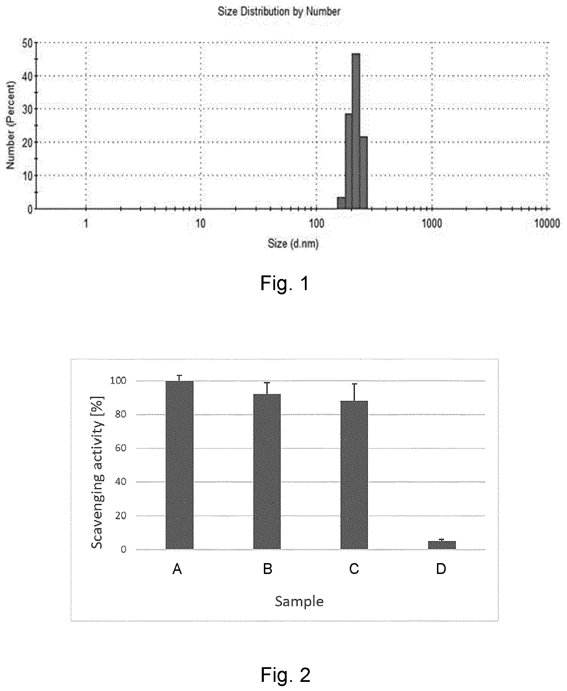

- Figure 1 shows a histogram graph of the average sizes of micro/nano capsules prepared according to the method of the invention, obtained by DLS analysis according to example 2. The graph shows on the x-axis the diameters (nm) of the micro/nano capsules analysed and on the y-axis the quantity of each population of micro/nano capsules measured.
- Figure 2 shows, in graphical form, the antioxidant activity (%), measured by DPPH assay according to example 3, of the control (A), of an astaxanthin solution dissolved in ethanol at a concentration of 50 µg/ml (B), of an encapsulated micro/nano astaxanthin suspension according to the invention at a concentration of 50 µg/ml (C) and of an empty micro/nano capsule suspension (D).

### DETAILED DESCRIPTION OF THE INVENTION

The Applicant has advantageously discovered that it is possible to produce an antioxidant action formulation for cosmetic use in which the antioxidant compounds contained therein are protected from the chemical and physical stresses due to the preparation, storage and distribution processes (shelf-life), so as to maintain the antioxidant functionality of these compounds unaltered, in addition to ensuring the controlled release over time of the aforesaid antioxidant compounds, by means of a method for producing micro/nano cosmetic capsules containing at least one antioxidant compound, comprising the following steps:
a) solubilising the antioxidant compound in an organic solvent;
b) mixing the solution obtained in step a) with an aqueous solution of lysozyme to obtain an antioxidant complex formed by the antioxidant compound and by the lysozyme;
c) evaporating at least a part of the organic solvent present in the solution obtained in step b);
d) mixing the solution obtained in step c) with an aqueous solution of hyaluronic acid;
e) homogenising the solution obtained in step d) to produce a homogeneous suspension of the antioxidant complex;
f) adding to the homogeneous suspension obtained in step e) an aqueous solution of a cross-linking compound and performing the homogenisation of the aqueous solution of the cross-linking compound in the aforesaid homogeneous suspension obtained in step e) to produce a plurality of micro/nano capsules containing the antioxidant compound.

Particularly, such micro/nano capsules obtained with the method of the invention are substantially formed by an antioxidant complex core, i.e., a core of antioxidant compound incorporated in lysozyme, and by a three-dimensional shell of hyaluronic acid and cross-linking compound enveloping said antioxidant complex.

Preferably but not necessarily, step c) of evaporating the organic solvent is achieved by mild stirring of the solution obtained in the previous step b).

The person skilled in the art is able to estimate how long it takes for the organic solvent to evaporate; this time may in fact depend on various factors, such as for example the amount of organic solvent used and the speed at which the solution is stirred.

Preferably, said step c) ends when substantially all of the organic solvent present in the solution has been removed by evaporation.

Evaporation of the organic solvent is allowed due to the particular complexation reaction between the lysozyme and the antioxidant compound particles. In fact, during step b), the antioxidant compound is incorporated into the three-dimensional structure of the lysozyme, producing a complex with the antioxidant compound which is thus stable in an aqueous environment. Optionally, steps b) and/or c) can be repeated in order to obtain different ratios of antioxidant compound to lysozyme, before proceeding to step d). Preferably, the antioxidant compounds used in the method of the present invention are extracted from microalgae and/or cyanobacteria, such as for example *Haematococcus pluvialis* and *Arthrospira platensis.*

Preferred examples not to be considered limiting of antioxidant compounds extracted from microalgae and/or cyanobacteria comprise astaxanthin and phycocyanin, which have high antioxidant and, in addition, anti-inflammatory properties, and are therefore very useful for cosmetic use.

Such compounds are in fact particularly advantageous when applied to the skin to achieve protection of the dermis from ultraviolet light and/or in the treatment of aesthetic imperfections of the dermis, such as for example acne and herpes.

The method of the present invention has proved particularly advantageous for the micro/nano encapsulation of poorly water-soluble or substantially water-insoluble antioxidant compounds.

The term "poorly water-soluble" means compounds in which the approximate volume of solvent (water) in ml per g of compound at a temperature of 25°C is higher than or equal to 100 ml. If this volume is greater than 10000 ml, the compound is understood to be "substantially insoluble".

According to the preferred embodiment of the method of the invention, the preferred antioxidant compound is astaxanthin.

As previously reported, astaxanthin is a carotenoid derived from the microalga *Haematococcus pluvialis* that can reduce oxidative stresses, preventing protein and lipid oxidation and DNA damage.

This compound, which is normally unusable due to its insolubility in water and its instability to heat and radiations, is protected from these stresses thanks to the micro/nano encapsulation method of the present invention, and, in addition, its antioxidant activity is preserved, as is shown in the results of example 3.

Further, as also reported earlier, the Applicants have advantageously found that mixing the aqueous solution containing astaxanthin with the aqueous solution of lysozyme achieves a very stable associative interaction between these two molecules, generating an antioxidant complex (also called host/guest complex) composed of these two molecules which are held together in a single structural relationship by bonds of a particularly weak type, such as for example hydrogen bonds, ionic coupling and/or Van der Waals forces, without the aid of oils or dedicated emulsions.

In addition, lysozyme also exhibits bacteriolytic and antiviral activity which is particularly advantageous in combination with the antioxidant activity of astaxanthin for use as a cosmetic on the skin.

Returning to the method according to the preferred embodiment of the invention, the preferred organic solvent is selected from acetone and ethanol.

It is not excluded that, according to alternative embodiments of the invention, the organic solvent is different from what is indicated.

According to the preferred embodiment of the method of the invention, astaxanthin is dissolved in the aforesaid organic solvent at a percentage concentration comprised between 0.01 and 10% by weight.

It should be noted that in this document, unless otherwise specified, the percentage concentrations reported are weight-to-weight percentage concentrations that correspond to the grams of solute dissolved in 100 g of solvent.

According to the preferred embodiment of the method of the invention, the aqueous solution of lysozyme has a lysozyme concentration comprised between 0.01 and 10% by weight.

Preferably, the aqueous solution of lysozyme is 0.2-3% by weight.

More preferably, the aqueous solution of lysozyme is at about 1% by weight. According to one aspect of the method of the invention, the preferred aqueous solution of lysozyme is a solution in which the solvent is a buffer solution adapted to maintain the pH at a value of about 7.5.

Preferably, but not necessarily, such an aqueous buffer solution is a solution of tris(hydroxymethyl)aminomethane hydrochloride having a pH of about 7.5.

It is not excluded that, according to alternative embodiments of the present invention, the concentrations of astaxanthin and/or lysozyme are different from what is indicated.

According to the preferred embodiment of the invention, the weight-to-weight ratio of hyaluronic acid:lysozyme is comprised between 4:1 and 1.25:1. Preferably, this ratio is 2:1.

Advantageously, the hyaluronic acid is able to bind to the skin's collagen and thus promotes the penetration of the content of the micro/nano capsules into the epidermis, i.e. it conveys the astaxanthin and lysozyme complexes contained in the micro/nano capsules into the epithelial tissue where they will perform their function, thus increasing the topical bioavailability of these compounds.

It cannot be ruled out that, according to alternative embodiments of the invention, this hyaluronic acid:lysozyme ratio is different from what is indicated. According to one aspect of the method of the invention, the preferred aqueous solution of hyaluronic acid is a solution in which the solvent is a buffer solution having a pH of about 4.

Returning to the preferred embodiment of the method of the invention, step e) and/or step f) of homogenization is carried out by high pressure homogenizer tool, or by magnetic stirring.

The use of homogenising tools results in a better distribution in the average sizes of the micro/nano capsules of hyaluronic acid containing astaxanthin and lysozyme.

In particular, the average sizes of the micro/nano capsules of hyaluronic acid obtained with the method according to the invention are comprised between 60 nm and 200 µm, preferably between 100 nm and 500 nm, more preferably between 200 nm and 300 nm.

The term "average sizes" refers to the average diameter of the micro/nano capsules, measured by DLS.

It is not excluded that, according to alternative embodiments of the invention, step e) and/or step f) can be carried out manually or with tools other than those indicated.

According to the preferred embodiment of the method of the invention, the homogenisation carried out in step f) advantageously allows to stabilise the polymeric hyaluronic acid shell thanks to the cross-linking compound which, by cross-linking with the polymer, stabilises the hyaluronic acid shell of the micro/nano capsule around the core of the astaxanthin-lysozyme antioxidant complex, preventing its separation.

The term cross-linking compound refers to a compound or mixture of compounds, either natural or synthetic, preferably of a polymeric type, whose chains rich in functional groups are adapted to react with different polymer chains creating covalent and non-covalent interactions, such as coordination and interaction bonds of opposite charges (coacerbation) that stabilise the chains involved in the bonds.

Preferably, the cross-linking compound added during step f) is selected from tetrapolyphosphate, calcium chloride, pectin, alginate, chitosan, lignosulfonates and natural gums.

Preferably, the natural gum is selected between xanthan gum and gum arabic. More preferably, such cross-linking compound is selected from chitosan and calcium chloride.

Even more preferably, the cross-linking compound is chitosan.

According to one aspect of the method of the invention, the weight-to-weight ratio of cross-linking compound/hyaluronic acid is comprised between 1:300 and 1:25.

Preferably, this ratio is about 1:100.

According to one aspect of the method of the invention, the aqueous solution of cross-linking compound is at 0.001-0.1% by weight.

It cannot be ruled out that, according to alternative embodiments of the present invention, the cross-linking compound is a compound other than what is indicated or is present in concentrations different from those reported above. Micro/nano capsules obtained by means of the method having the features described for the preferred embodiment of the invention, including variants, are also part of the present invention.

Such micro/nano capsules are particularly suitable for use as a cosmetic for treating the skin of a subject, preferably a human being.

Advantageously, such micro/nano capsules are suitable for use in the field of dermocosmetics, in particular in the method for treating acne and herpes.

Still advantageously, the aforesaid micro/nano capsules are in the form of an antioxidant formulation for cosmetic use.

It is specified that, for the cosmetic formulation of the invention, when reference is made to the following products: antioxidant compound, antioxidant complex, organic solvent and cross-linker, these products are to be considered to have the same characteristics as the products identified by the same name in the description of the method according to the invention proposed above, including variants.

According to a preferred aspect of the cosmetic formulation of the present invention, it comprises a plurality of micro/nano capsules obtainable according to the method of the invention defined above for the preferred embodiment of the invention, including the variants.

Preferably, such micro/nano capsules contain at least the antioxidant complex formed by astaxanthin and lysozyme.

Further preferably, the shells of such micro/nano capsules comprise a combination of hyaluronic acid and at least one cross-linking compound.

Such cross-linking compound is preferably selected from chitosan and calcium chloride.

According to a preferred aspect of the cosmetic formulation of the invention, it may be made in the form of a gel, ointment, cream, pomade, foam, powder, spray, oil-in-water emulsion, water-in-oil emulsion, oil-in-gel emulsion, gel-in-oil emulsion, suspension, or any other cosmetic form suitable for topical administration.

According to one aspect of the cosmetic formulation of the present invention, the formulation is in the form of a cream or gel, preferably the aforesaid formulation is in the form of a cream.

The cosmetic formulation of the present invention may comprise one or more excipients of pharmaceutically acceptable grade.

Preferably, such excipients are pharmaceutically acceptable excipients for topical use.

Such excipients are excipients of a type known to the person skilled in the art and may comprise, among others, carriers, diluents, excipients, emulsifiers, emollients, preservatives, consistency factors, pH adjusters.

According to one aspect of the present invention, the cosmetic formulation can be contained in a medical device for the application of cosmetics topically.

In particular, such a device for the application of cosmetics topically is a device of the type configured to adhere to the body of a subject at least during use. The subject is preferably a human being.

By way of example not to be considered limiting, such a device may be a medicated, adhesive or non-adhesive film, a medicated plaster, a medicated gauze, a medicated bandage, a medicated patch, to be applied at least on the area of the skin to be treated with the aforesaid cosmetic formulation.

It is specified that the term "medicated" in this description means that the film, the plaster, the gauze, the bandage or the patch comprise the cosmetic formulation according to the present invention, for example a plaster soaked in a pomade, ointment or cream of micro/nano capsules of the present invention. The aforesaid device for the application of cosmetics topically, comprising a plurality of micro/nano capsules obtained with the method of the present invention or comprising the cosmetic formulation of the present invention, is therefore also part of the present invention.

According to one aspect of the present invention, such a device is preferably produced by three-dimensional printing.

Three-dimensional printing makes it possible to produce a customised device based on the subject and the area of the subject to be treated. Advantageously, the cosmetic formulation of the present invention can be mixed with a composition for three-dimensional printing, so-called printing ink, in such a way that, during the manufacturing process of the device, the formulation of the invention is incorporated into the composition of the device. By way of example not to be considered limiting, the formulation of the present invention may be mixed with a polymer resin for printing by stereolithography. Therefore, the use of the cosmetic formulation as defined above, including the variants, for the production by three-dimensional printing of the aforesaid device for the application of cosmetics topically comprising such formulation is also part of the present invention.

Further characteristics and advantages of the method and formulation of the invention will become apparent to the person skilled in the art from the examples below which are provided for the purpose of a better understanding of what is described and are not intended as limitations of the claims.

### EXAMPLES

### Example 1. Production of micro/nano capsules with the method of the present invention.

5 mg of astaxanthin were dissolved in 5 ml of acetone and suitably mixed. The solution thus obtained has a concentration of 0.1%.

100 mg of lysozyme were also dissolved in 100 ml of a solution of tris(hydroxymethyl)aminomethane and the pH raised to the value of 7.5 by addition of hydrochloric acid.

1 ml of 0.1% astaxanthin solution was added to the 0.1% lysozyme solution and gently stirred in order to make the two molecules interact and achieve simultaneous evaporation of the organic solvent.

This operation was repeated for all 5 ml of astaxanthin solution.

100 ml of a solution of hyaluronic acid at 0.2% concentration in acetate buffer at pH 4.5 were also prepared and slowly added to the lysozyme-astaxanthin complex solution, under magnetic stirring.

Finally, 20 ml of a cross-linking solution of calcium chloride, at 0.01% concentration, were slowly added and homogenized to the suspension of astaxanthin-lysozyme-hyaluronic acid.

### Example 2. Size analysis of the micro/nano capsules obtained in example 1 by Dynamic Light Scattering (DLS).

The DLS analyses were carried out by diluting the samples obtained according to example 1 in 1/1000 of milliQ water, so as to obtain the optimal opening of the shutter and ensure maximum reliability of the analysis. 800 µl of each sample was placed in a plastic cuvette and inserted into the Zetasizer NanoZS instrument (Malvern) for performing the measurement. Each measurement was performed in triplicate.

The results obtained are shown in Figure 1 where it can be seen that the average sizes of the micro/nano capsules obtained were around 250 nm.

### Example 3. DPPH assay for the evaluation of the antioxidant activity of micro/nano capsules of astaxanthin.

To evaluate and compare the antioxidant activity of astaxanthin encapsulated by means of the method of the invention and astaxanthin in unencapsulated free form, the DPPH assay was performed. This assay makes it possible to determine the antioxidant power of a sample by reacting it with a solution of DPPH (2,2-diphenyl-1-picrylhydrazyl) and analysing, by means of a UV-Vis spectrophotometer, the decay of the diphenylpicrylhydrazyl radical in the absence or presence of the sample, after a predetermined incubation time.

In detail, the reaction was carried out on 700 µl plastic cuvettes using a solution containing ethanol as a blank.

A stock of 50 ml of 0.5 mM DPPH in ethanol was initially prepared.

The samples to be analysed on the spectrophotometer as follows were also prepared: 20 µl of sample (B, C or D) + 560 µl of DPPH stock solution + 20 µl of ethanol.

The samples comprised:
A) the control sample corresponding to the solution of 560 µl of DPPH stock solution + 40 µl of ethanol;
B) a solution of astaxanthin dissolved in ethanol at a concentration of 50 µg/ml;
C) an encapsulated suspension of astaxanthin at a concentration of 50 µg/ml;
D) a suspension of empty micro/nano capsules (formed by lysozyme and hyaluronic acid) at concentrations equivalent to those of sample C.

The samples were stirred and placed out of the light for about 4 hours. The absorbance of the sample was then read at 517 nm, normalizing it for the absorbance values of the blank.

The results obtained, represented in Figure 2, show how the astaxanthin contained in the micro/nano capsules has an antioxidant capacity comparable to the free-form astaxanthin sample, demonstrating that the micro/nano capsules obtained with the method of the invention protect astaxanthin from the external environment and, at the same time, maintain its antioxidant function unaltered.

Therefore, based on the above, the present invention has achieved all of the predetermined objects.

In particular, the object of providing a new method for producing cosmetic formulations comprising antioxidant compounds of natural origin, in particular antioxidants extracted from microalgae and/or cyanobacteria, is achieved.

In addition, this method makes it possible to produce formulations in which the antioxidant compounds present in them are protected from environmental stresses, such as heat, light radiation and, in some cases, the presence of oxygen and, at the same time, maintain their antioxidant activity.

Again, thanks to the hyaluronic acid/cross-linker combination, the antioxidant compounds present in such formulations exhibit excellent bioavailability, particularly for topical use.

Last but not least, a cosmetic formulation particularly suitable for use as a topical cosmetic is made with the method of the present invention.

## Claims

1. Method for producing cosmetic micro/nano capsules, said micro/nano capsules containing at least one antioxidant compound, **characterized by** comprising the following steps:
a) solubilising said antioxidant compound in an organic solvent;
b) mixing the solution obtained in step a) with an aqueous solution of lysozyme to obtain an antioxidant complex formed by said antioxidant compound and by said lysozyme;
c) evaporating at least a part of the organic solvent present in the solution obtained in step b);
d) mixing the solution obtained in step c) with an aqueous solution of hyaluronic acid;
e) homogenising the solution obtained in step d) to produce a homogeneous suspension of said antioxidant complex;
f) adding to said homogeneous suspension obtained in step e) an aqueous solution of a cross-linking compound and performing the homogenisation of said aqueous solution of a cross-linking compound in said homogeneous suspension obtained in step e) to produce said micro/nano capsules containing said antioxidant compound.

2. Method according to the preceding claim, **characterized in that** said antioxidant compound is poorly water-soluble or substantially insoluble in water.

3. Method according to any one of the preceding claims, **characterized in that** said antioxidant compound is a compound extracted from microalgae and/or cyanobacteria.

4. Method according to the preceding claim, **characterized in that** said antioxidant compound is astaxanthin.

5. Method according to any one of the preceding claims, **characterized in that** said aqueous solution of lysozyme is 0.2-3% by weight.

6. Method according to any one of the preceding claims, **characterized in that** the weight-to-weight ratio of hyaluronic acid:lysozyme is comprised between 4:1 and 1.25:1, preferably it is 2:1.

7. Method according to any one of the preceding claims, **characterized in that** said cross-linking compound is selected from tetrapolyphosphate, calcium chloride, pectin, alginate, chitosan, lignosulfonates, natural gums, preferably said cross-linking compound is selected from chitosan and calcium chloride.

8. Method according to any one of the preceding claims, **characterized in that** the weight-to-weight ratio of cross-linking compound/hyaluronic acid is comprised between 1:300 and 1:25, preferably it is 1:100.

9. Method according to any one of the preceding claims, **characterized in that** said micro/nano capsules have average sizes comprised between 60 nm and 200 pm, preferably between 100 nm and 500 nm, more preferably between 200 nm and 300 nm.

10. Cosmetic formulation comprising a plurality of micro/nano capsules obtainable according to the method defined in any one of claims 1 to 9 and optionally at least one excipient of pharmaceutically acceptable grade, **characterized in that** said micro/nano capsules comprise a core of at least one antioxidant complex formed by lysozyme and astaxanthin, the shell of said micro/nano capsules comprising a combination of hyaluronic acid and at least one cross-linking compound.

11. Formulation according to the preceding claim, **characterized by** being in the form of a gel, ointment, cream, pomade, foam, powder, spray, oil-in-water emulsion, water-in-oil emulsion, oil-in-gel emulsion, gel-in-oil emulsion, suspension or by being contained in a device for the application of cosmetics topically.

12. Device for the application of cosmetics topically, said device being adapted to adhere to the body of a subject at least during use, said device comprising a cosmetic formulation as defined in any one of claims 10 or 11.

13. Use of the cosmetic formulation as defined in claim 10 for producing, by three-dimensional printing, a device for the application of cosmetics topically comprising said formulation.
